# EUROPEAN PATENT SPECIFICATION

(11) **EP 2 231 062 B1**
(45) Date of publication and mention of the grant of the patent: **02.03.2016**
(21) Application number: 08855777.2
(22) Date of filing: 02.12.2008
(51) Int. Cl.: A61F 2/00

(54) **IMPLANT FOR PARASTOMAL HERNIA**
IMPLANTAT FÜR EINE PARASTOMALE HERNIE
IMPLANT POUR HERNIE PARASTOMALE

(30) Priority: 03.12.2007 FR 0708429; 03.12.2007 US 5131
(43) Date of publication of application: 29.09.2010
(60) Divisional application: 15194577.1
(73) Proprietor: SOFRADIM PRODUCTION, 01600 Trévoux (FR)
(72) Inventor: SPINNLER, Linda, F-69480 Pommiers (FR); LECUIVRE, Julie, 69620 Le Bois d'Oingt (FR); MENEGHIN, Alfredo, F-69009 Lyon (FR); THERIN, Michel, F-69004 Lyon (FR)
(74) Representative: Verriest, Philippe
(86) International application number: PCT/IB2008/003761
(87) International publication number: WO 2009/071998

(56) References cited:
- WO-A-00/67663
- WO-A-2004/071349
- WO-A-2004/103212
- US-A1- 2003 212 460

## Description

The present invention relates to an implant suitable for use in the prevention and/or treatment of hernias that may occur in the area of a stoma, particularly one formed in the abdominal wall.

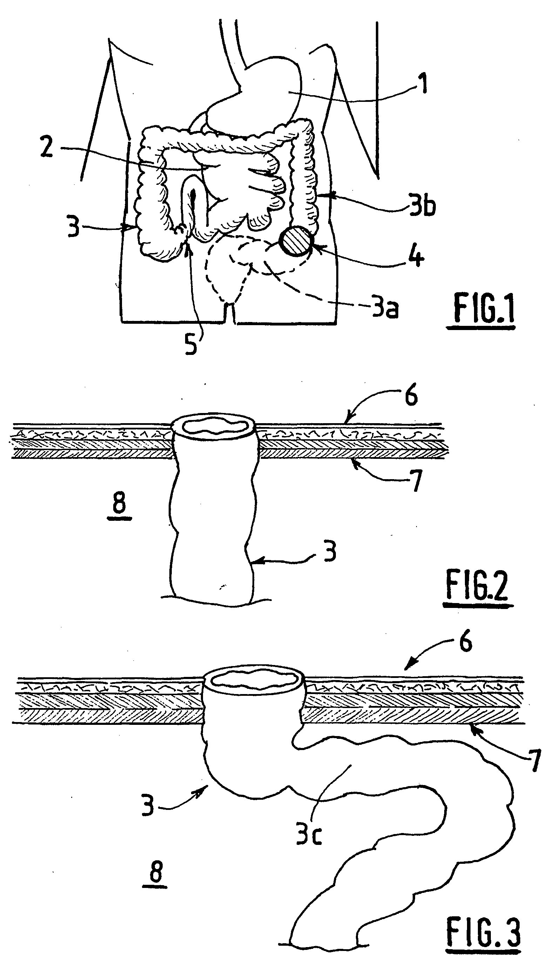

Stomas are openings formed in a wall, for example the abdominal wall, for joining a hollow organ, for example the intestine, to the skin. Such an operation proves necessary, for example in cases of cancer of the rectum or Crohn's disease, to create an artificial anus for example, during which operation the diseased part of the intestine is resected and the healthy intestine is exteriorized at the skin. In this case, the stoma is formed in the abdominal wall. Figure 1 is a schematic illustration of the human digestive tract. This diagram shows the stomach 1, the small intestine 2 and the colon 3. The broken lines represent the part 3a of the colon that is diseased and has been removed during the surgical procedure. The healthy part 3b of the colon now opens to the outside at the stoma 4 formed in the abdominal wall. Depending on the extent of the diseased part of the colon, the stomas can be formed in the area of the ileum 5 (ileostomy) or of the colon (colostomy), as shown in Figure 1.

Stomas can also be formed in the area of the ureters (ureterostomy).

After operations of this kind, hernias may develop around the stoma, that is to say in the area of the peristomal wall. A weakening of the wall around the stoma may therefore result in the appearance of a parastomal hernia. To treat these parastomal hernias, prostheses are implanted that are designed to strengthen the abdominal wall inside the patient, in the area of the stoma. The implantation of these prostheses can be intraperitoneal, that is to say within the actual abdominal wall, or retroperitoneal, resting against the abdominal wall.

Prostheses for treating parastomal hernias have been described in the document WO2004/071349. However, these prostheses are not entirely satisfactory, particularly since they are not adapted to all types of stomas that are formed, particularly indirect stomas.

The reason is that, for example in the case of the colon, several stoma configurations can be formed: the direct stoma, as shown in Figure 2, in which the colon 3 issuing from the abdominal cavity 8 is perpendicular to the abdominal wall 7 and hence to the skin 6 prior to its exteriorization, or the indirect stoma, as shown in Figure 3, in which the colon 3 is caused to form a bend within the abdominal cavity 8 prior to its exteriorization, the colon thus having a part 3c parallel to the abdominal wall 7. The indirect stoma avoids a situation where the internal part of the colon in the area of the stoma becomes invaginated and exteriorizes.

Other prosthesis are described in documents WO2004/103202 and in US 2003/212460.

There is therefore a need for a parastomal prosthesis able to protect the intestine and hollow organs and to effectively strengthen the abdominal wall regardless of the type of stoma that has been formed.

The present invention aims to meet this need by making available an implant that has specific surfaces able to protect the hollow organs, such as the intestine, regardless of the stoma that has been formed, and at the same time to effectively strengthen the abdominal wall.

The subject matter of the present invention is an implant for the prevention or treatment of a hernia formed in the abdominal wall in the proximity of a stoma of an organ, comprising a layer of a porous structure whose surface intended to face the abdominal cavity is covered by a first film of anti-adhesive material, characterized in that said porous structure comprises a first part intended to be in contact with the stoma organ and having a first thickness E1, and a second part having a second thickness E2 greater than said first thickness E1, said first part being covered, on its surface intended to face the abdominal wall, by a second film of anti-adhesive material.

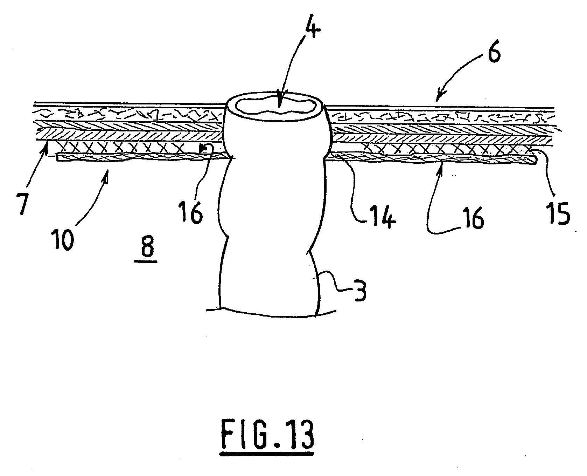

Thus, in the implant according to the invention, the first part of the porous structure, the part intended to be in contact with the stoma organ, for example in contact with the intestine, is covered by a film of anti-adhesive material on both of its surfaces. In one embodiment of the invention, the first and second films of anti-adhesive material are joined to form just one film, and the first part of porous structure is totally enclosed within the film of anti-adhesive material. As will become clear from the explanations given later with reference to Figures 13 to 15, the stoma organ, for example the intestine, is protected irrespective of whether the stoma is a direct or indirect one, because the part of the implant able to come into contact with it is covered by a film of anti-adhesive material.

In the present application, an "implant" is understood as a biocompatible medical device that can be implanted in the human or animal body.

Within the meaning of the present application, the word "porous" is understood as the characteristic according to which a structure has pores or meshes, pockets, holes or orifices, that are open and are distributed uniformly or irregularly and promote all cell colonization. The pores can be present in all types of configurations, for example as spheres, channels, hexagonal forms.

According to one embodiment of the invention, the porous structure comprises a sponge, a fibrous matrix or a combination of a sponge and of a fibrous matrix. For example, the sponge can be obtained by lyophilization of a gel, with pores being created during the lyophilization. The fibrous matrix can be any arrangement of yarns or yarn portions creating pores between the yarns and/or yarn portions. For example, the fibrous matrix can be a textile, for example obtained by knitting or weaving or according to a technique for producing a nonwoven.

In one embodiment of the invention, the porous structure, for example the sponge and/or the fibrous matrix, has pores with dimensions ranging from approximately 0.1 to approximately 3 mm.

In one embodiment of the invention, the porous structure comprises a textile. For example, the porous structure can be composed of a textile.

According to one embodiment of the invention, said thickness E1 of the first part of the porous structure ranges from approximately 0.15 to 0.50 mm. A relatively small thickness of this kind allows the abdominal wall to be strengthened without any risk of damaging the stoma organ, for example the intestine, which is in contact with the implant.

Said first part of porous structure is preferably a textile in the form of a knit. This knit is preferably a two-dimensional knit, that is to say preferably a knit having a thickness less than or equal to 5 times the mean diameter of the yarns from which it is made, for example knitted on a warp knitting machine or raschel machine with the aid of two guide bars forming a knit with two surfaces, said knit being free of sheets of connecting yarns between its two opposite surfaces.

When the first part of porous structure is a two-dimensional knit as defined above, the pores are formed by the empty spaces situated between the constituent yarns of the knit, for example the meshes.

The constituent yarns of the knit that form the first part of porous structure can be chosen from among yarns made of biocompatible materials, bioabsorbable materials, non-bioabsorbable materials and their mixtures.

In the present application, the word "bioabsorbable" is understood as the characteristic according to which a material is absorbed by the biological tissues and disappears *in vivo* at the end of a given period, which can vary for example from one day to several months, depending on the chemical nature of the material.

Thus, examples of bioabsorbable materials suitable for the yarns forming the first part of porous structure are polylactic acid (PLA), polysaccharides, polycaprolactones (PCL), polydioxanones (PDO), trimethylene carbonates (TMC), polyvinyl alcohol (PVA), polyhydroxyalkanoates (PHA), polyamides, polyethers, oxidized cellulose, polyglycolic acid (PGA), copolymers of these materials and their mixtures.

Examples of non-bioabsorbable materials suitable for the yarns forming the first part of porous structure are polypropylenes, polyesters such as polyethylene terephthalates, polyamides, polyvinylidene fluoride, and their mixtures.

The yarns forming the first part of porous structure of the implant can, for example, be chosen from among monofilament yarns, multifilament yarns and their combinations.

The multifilament yarn count preferably varies from 40 to 110 dtex.

The monofilament yarns preferably have a diameter ranging from 0.06 to 0.15 mm.

In one embodiment of the invention, the yarns forming the first part of porous structure are monofilament yarns. Such monofilament yarns pose less risk of sepsis than do multifilament yarns.

In one embodiment of the invention, the monofilament yarns are of polyethylene terephthalate.

A monofilament yarn suitable for the first textile part of the implant according to the invention is, for example, a monofilament yarn with a diameter of approximately 0.08 mm, of polyethylene terephthalate.

The porous structure of the implant according to the invention comprises a second part with a thickness E2 greater than the thickness E1 of the first part. The second part of porous structure is essentially designed to act as a reinforcement of the abdominal wall.

Thus, the value of the thickness E2 of the second part of porous structure can vary depending on the value of the thickness E1 of the first part of the structure, the value of the thickness E2 of the second part of porous structure needing to be greater than that of the value of the thickness E1 of the first part of porous structure. The reason is that the second part of porous structure preferably has mechanical strength superior to that of the first part of porous structure. For example, said second thickness E2 of the second part of the porous structure can range from approximately 0.40 to 3.00 mm.

As will become clear from the description that follows, the surface of the layer of porous structure intended to be placed facing the abdominal cavity is covered by a film of anti-adhesive material which prevents the organs and other viscera of the abdominal cavity from attaching themselves to the implant. This surface will be referred to hereinafter as the closed surface of the implant. By contrast, the surface of the second part of porous structure intended to be placed facing the abdominal wall is not covered by a film of anti-adhesive material and remains open to all cell colonization at the time of implantation. This surface will be referred to hereinafter as the open surface of the second part of porous structure. This surface of the second part of porous structure is intended to be placed resting against the abdominal wall. To permit better fixing of the implant to the abdominal wall, the open surface of the second part of porous structure can comprise fastening means, for example self-fixing ones, inherent to this surface. Thus, by virtue of its porous character and its thickness, the second part of porous structure of the implant according to the invention is especially adapted to promote tissue growth via its open surface after implantation. The cells of the abdominal wall deeply colonize the second part of porous structure by way of its open surface placed facing the abdominal wall.

In one embodiment of the invention, the second part of porous structure is a textile in the form of a three-dimensional knit, for example as described in applications WO99/06080 and WO99/05990. Within the meaning of the present application, the term "three-dimensional knit" is understood as an assembly or arrangement of monofilament or multifilament yarns or a combination of these, obtained by knitting and having two opposite surfaces that are separated by a significant thickness, preferably of greater than or equal to 0.50 mm, said thickness comprising connecting yarns and pores.

Such a three-dimensional knit can be knitted, for example, on a warp knitting machine or double-bed raschel machine with the aid of several guide bars forming a knit that comprises two opposite surfaces and a spacer. In the present application, the word "spacer" is understood as the set or sets of yarns that connect the two surfaces of a three-dimensional knit to each other, thereby constituting the thickness of a knit, as is described in WO99/06080 or in WO99/05990.

Thus, in the case where the second part of porous structure is a three-dimensional knit as described above, the knitting structure can define within the thickness of the knit a multiplicity of transverse channels or pockets that may or may not be mutually parallel. These pockets or channels can be interconnected and thus allow the colonizing cells to pass from one pocket or channel to another. A second part of porous structure of this type promotes good tissue growth after implantation.

The yarns constituting the second part of porous structure of the implant according to the invention can be chosen from among yarns made of biocompatible materials, bioabsorbable materials, non-bioabsorbable materials and their mixtures, already listed above for the first part of porous structure.

Thus, examples of bioabsorbable materials suitable for the yarns forming the second part of porous structure are polylactic acid (PLA), polysaccharides, polycaprolactones (PCL), polydioxanones (PDO), trimethylene carbonates (TMC), polyvinyl alcohol (PVA), polyhydroxyalkanoates (PHA), polyamides, polyethers, oxidized cellulose, polyglycolic acid (PGA), copolymers of these materials and their mixtures.

Examples of non-bioabsorbable materials suitable for the yarns forming the second part of porous structure are polypropylenes, polyesters such as polyethylene terephthalates, polyamides, polyvinylidene fluoride, and their mixtures.

The yarns forming the second part of the porous structure can, for example, be chosen from among monofilament yarns, multifilament yarns and their combinations.

The multifilament yarn count preferably varies from 40 to 110 dtex.

The monofilament yarns preferably have a diameter ranging from 0.06 to 0.15 mm.

In one embodiment of the invention, the yarns forming the first part of porous structure are monofilament yarns. Such monofilament yarns pose less risk of sepsis than do multifilament yarns. For example, the monofilament yarns are of polyethylene terephthalate.

A monofilament yarn suitable for the second part of porous structure of the implant according to the invention is, for example, a monofilament yarn with a diameter of approximately 0.08 mm, of polyethylene terephthalate.

In one embodiment of the invention, said second part of porous structure has, on its open surface intended to face the abdominal wall, means of fastening said second part to said abdominal wall. These fastening means can be chosen from among elements that are integrally formed on said second textile part, such as loops and barbs, or from among elements joined to the surface of the second textile part, such as a rough covering, hooks, threads or clips fixed on the surface of the second textile part.

In one embodiment of the invention, said fastening means are chosen from among loops, barbs and their mixtures. In such a case, the loops and barbs can be obtained from yarns or portions of yarns that are woven and/or knitted directly for example, with the three-dimensional knit forming the second part of porous structure. For example, in order to obtain barbs, it is possible to use hot-melt yarns such as are described in the application WO01/81667.

In the embodiment of the invention in which the first part of porous structure is in the form of a two-dimensional knit and the second part of porous structure is in the from of a three-dimensional knit, the two knits, i.e. two-dimensional and three-dimensional, can be manufactured separately then joined together by at least one seam, for example, in order to form the layer of porous structure of the implant.

In another embodiment, the two-dimensional knit and the three-dimensional knit are knitted together on the same knitting machine and constitute a textile made in one piece, for example by using supplementary guide bars for the three-dimensional knit and/or different yarn runs for producing each of the two knits. In such an embodiment of the invention, the porous structure layer of the implant according to the invention is composed of a textile formed in one piece, said textile having a two-dimensional zone, corresponding to the first part of the porous structure, and one or more three-dimensional zones, corresponding to the second part of the porous structure. In such an embodiment, it is possible to form a selvage at the passage from a two-dimensional zone to a three-dimensional zone with a view to forming a smooth connection between the two parts, such that the difference in thickness between the two parts does not form a step that could damage the biological tissue situated in the proximity of the implant.

The layer of porous structure of the implant according to the invention is covered, on its second surface intended to face the abdominal cavity, by a first film of anti-adhesive material. Moreover, the first part of porous structure is covered, on its surface intended to face the abdominal wall, by a second film of anti-adhesive material.

Within the meaning of the present application, the term "anti-adhesive material" is understood as a smooth and non-porous biocompatible material that prevents the organs and other viscera of the abdominal cavity from attaching themselves to the implant.

The anti-adhesive material forming the first film can be identical to or different from the material forming the second film.

In one embodiment of the invention, the anti-adhesive material constituting the first and/or second film(s) is chosen from among bioabsorbable materials, non-bioabsorbable materials and their mixtures.

In one embodiment of the invention, the bioabsorbable materials suitable for the first and/or second film(s) of anti-adhesive material are chosen from among collagens, oxidized celluloses, polyarylates, trimethylene carbonates, caprolactones, dioxanones, glycolic acid, lactic acid, glycolides, lactides, polysaccharides, for example chitosans, polyglucuronic acids, hylauronic acids, dextrans and their mixtures.

In one embodiment of the invention, the non- bioabsorbable materials suitable for the first and/or second film of anti-adhesive material are chosen from among polytetrafluoroethylene, polyethylene glycols, polysiloxanes, polyurethanes, stainless steels, derivatives of precious metals and their mixtures.

In one embodiment of the invention, the material constituting the first and/or second film(s) of anti-adhesive material is a hydrophilic bioabsorbable material, preferably chosen from the group formed by collagens, polysaccharides and their mixtures. Of the collagens that can be used according to the invention, the following may be mentioned:
1) collagen whose helical structure is at least partially denatured by heat, without hydrolytic degradation, and whose method of preparation is described in WO99/06080,
2) native collagen, not heated, filmed with or without glycerol, crosslinked by gamma irradiation or by other chemical or physical means,
3) and/or their mixtures.

Of the polysaccharides that can be used as absorbable hydrophilic material according to the invention, the following may be mentioned: oxidized cellulose, hylauronic acid, starch, chitosan, crosslinked dextrans and/or their mixtures. All these materials are well known to persons skilled in the art. An oxidized cellulose suitable for the present invention is the product sold under the brand name "Interceed^{®}" by Ethicon. A hyaluronic acid suitable for the present invention is the product sold under the brand name "Hyalobarrier^{®}" by Fidia Advanced Biopolymers, or the product sold under the brand name "Seprafilm^{®}" by Genzyme.

In one embodiment of the invention, the first film and the second film form a single and unique film, the first film then completely coating the first part of porous structure and thus covering this porous structure part both on its surface intended to face the abdominal cavity and also on its surface intended to face the abdominal wall. Thus, said first part of porous structure is totally enclosed in the film of anti-adhesive material before implantation and at the moment of implantation.

Thus, at the moment of implantation, and whatever the embodiment of the invention, the two surfaces of the first part of porous structure are occluded by a continuous film of anti-adhesive material.

The first part of the porous structure of the implant according to the invention, regardless of whether it is totally coated by the first film of anti-adhesive material or whether each of its surfaces are covered, one by the first film of anti-adhesive material, the other by the second film of anti-adhesive material, is thus protected at least during the initial phase of cicatrization, i.e. is not exposed to the inflammatory cells such as granulocytes, monocytes, macrophages, or the multinucleated giant cells that are generally activated by the surgical procedure. Nor is it exposed to the bacteria that may be present. The reason for this is that, at least during the initial phase of cicatrization, which may last approximately 5 to 10 days, only the film or films of anti-adhesive material are accessible to the various factors such as proteins, enzymes, cytokines or inflammatory cells, in the first textile part.

In the case where the film or films of anti-adhesive material are made of non-absorbable materials, they thus protect the first part of porous structure before and after implantation, throughout the period of implantation of the implant.

Furthermore, by virtue of the film or films of anti-adhesive material, the surrounding fragile tissues, such as the hollow viscera for example, are protected in particular from the formation of severe postsurgical fibrous adhesions.

In the case where the anti-adhesive material comprises a bioabsorbable material, it is preferable to choose a bioabsorbable material that is not absorbed until after a few days, such that the film of anti-adhesive material can perform its function of protecting the stoma organ, for example the intestine, and the hollow organs during the days following the operation, and until the cellular recolonization of the implant in turn protects the fragile organs.

The thickness of the first anti-adhesive film is preferably much less than the thickness E2 of the second part of porous structure. In fact, the film of anti-adhesive material must preferably not occlude the open surface of the second part of the porous structure, so as to permit cellular recolonization of the second part of porous structure after implantation.

The first film of anti-adhesive material is preferably continuous, smooth and non-porous, covering the whole surface of the porous structure intended to be placed facing the abdominal cavity. In one embodiment, the first film of anti-adhesive material extends past the edges of the layer of porous structure. Thus, the implant is protected from contact with the viscera. The first film of anti-adhesive material can, for example, extend past the edges of the layer of porous structure by a distance ranging from 3 to 10 millimetres.

The first film of anti-adhesive material is preferably joined to the surface of the layer of porous structure intended to be placed facing the abdominal cavity by means of surface penetration, keeping open the porosity on the opposite surface of the second part of the porous structure, that is to say the open surface, intended to be placed facing the abdominal wall.

The implant according to the invention can be used via the laparoscopic route. If necessary, for example when the first and second films of anti-adhesive material are made of dried collagen, it is preferable to rehydrate the implant at the time of use, in order to make it flexible and easier to use.

The implant according to the invention can, for example, be prepared according to the following method:
a) a textile is prepared that has two-dimensional zones and three-dimensional zones, as has been described above,
b) a solution of an anti-adhesive material is prepared,
c) the solution obtained at b) is poured into a mould,
d) the textile is then applied to the solution, the surface of the textile intended to face the abdominal cavity being placed on said solution in such a way that said solution impregnates the two-dimensional zones of said textile completely,
e) it is left to dry.

With such a method it is possible to obtain an implant according to the invention in which the first film and the second film form a single and unique film.

Alternatively, step d) is replaced by step d') in which the solution of anti-adhesive material only superficially impregnates a single surface of the two-dimensional zones, thereby forming the first film. The procedure is then supplemented by an additional step in which the opposite surface of the two-dimensional zones is impregnated by the same solution of anti-adhesive material or by another solution of another anti-adhesive material in order to form the second film.

Methods of covering/coating that can be used according to the present invention are described in documents WO99/06080 and WO2004/043294.

The implant according to the invention can have any shape adapted to the anatomy of the patient and/or to the surgical technique envisaged. For example, the shape of the implant can be round, oval, square or rectangular.

In one embodiment, the implant has a generally elongate shape, for example oval or rectangular. For example, the length of the implant can range from 12 to 30 cm and its width can range from 10 to 20 cm.

In another embodiment, the implant has a generally round shape. For example, the diameter of the implant can range from 5 to 20 cm.

In one embodiment of the invention, said first part of porous structure has the form of a central strip, and, for example, the width of the central strip can range from 2 to 10 cm.

In another embodiment of the invention, said first part of porous structure has the form of a disc, and, for example, the diameter of the disc can range from 2 to 10 cm.

In one embodiment of the invention, at least one orifice is formed at the centre of the first part of the porous structure in order to provide a passage for the stoma organ, for example the intestine, during implantation of the implant. Alternatively, at least one orifice is formed within the first part of the porous structure, said orifice being offset relative to the centre of the implant. For certain types of surgery, for example ureterostomies, the implant can have two orifices. In one embodiment of the invention, the orifice or orifices can be connected to an edge of the implant by way of a slit. For example, the dimensions of the orifices can range from 0.5 to 8 cm. The orifice or orifices can be offset relative to the centre of the implant.

The advantages of the present invention, and variants thereof, will become evident from the following detailed description and from the attached drawings, in which:
Figure 1 is a schematic illustration of the human digestive tract, in which a stoma has been formed,
Figure 2 is a schematic illustration of a direct stoma,
Figure 3 is a schematic illustration of an indirect stoma,
Figure 4 is a plan view of a first embodiment of an implant according to the invention,
Figure 5 is a plan view of a second embodiment of an implant according to the invention,
Figure 6 is a simplified schematic cross-sectional view of the implant from Figure 4,
Figure 7 is a photograph taken with a Hitachi S-800 FEG scanning electron microscope, magnification x40, showing an embodiment of the first part of porous structure of an implant according to the invention,
Figure 8 is a photograph taken with a Hitachi S-800 FEG scanning electron microscope, magnification x250, showing the first part of porous structure from Figure 7 once enclosed in the film of anti-adhesive material,
Figure 9 is a photograph taken with a Hitachi S-800 FEG scanning electron microscope, magnification x20, showing an embodiment of the second part of porous structure of an implant according to the invention, covered on one surface by the first film of anti-adhesive material,
Figures 10, 10A, 11 and 12 show embodiments of the knitting structure suitable for producing a textile for an implant according to the invention,
Figure 13 is a cross-sectional view of an implant according to the invention once it has been implanted after a direct colostomy,
Figure 14 is a schematic plan view of another embodiment of an implant according to the invention once it has been implanted after an indirect colostomy,
Figure 15 is a cross-sectional view of the implant from Figure 14 along the line II in Figure 14.

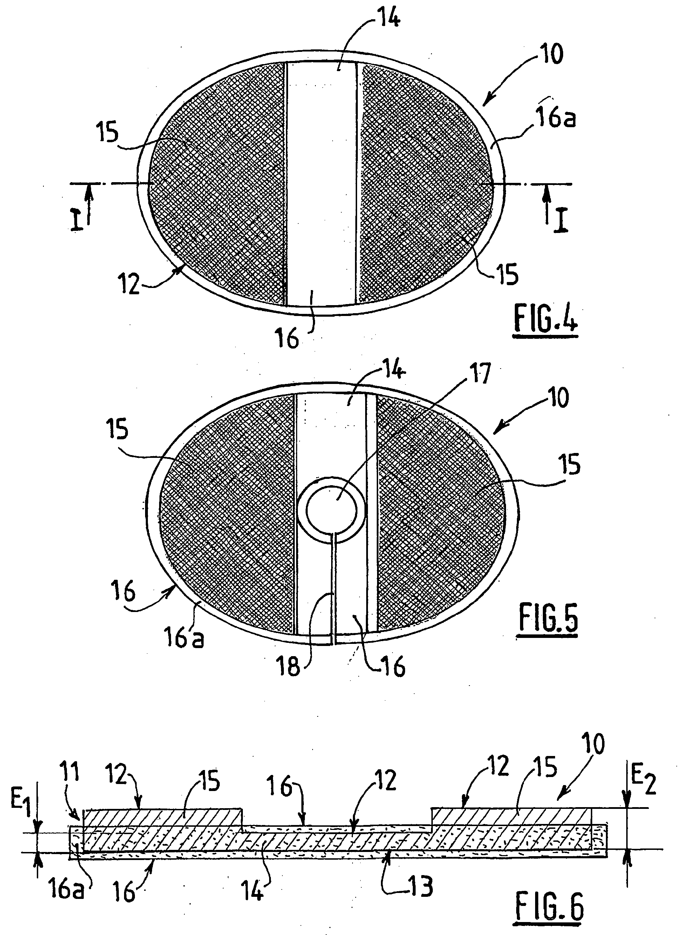

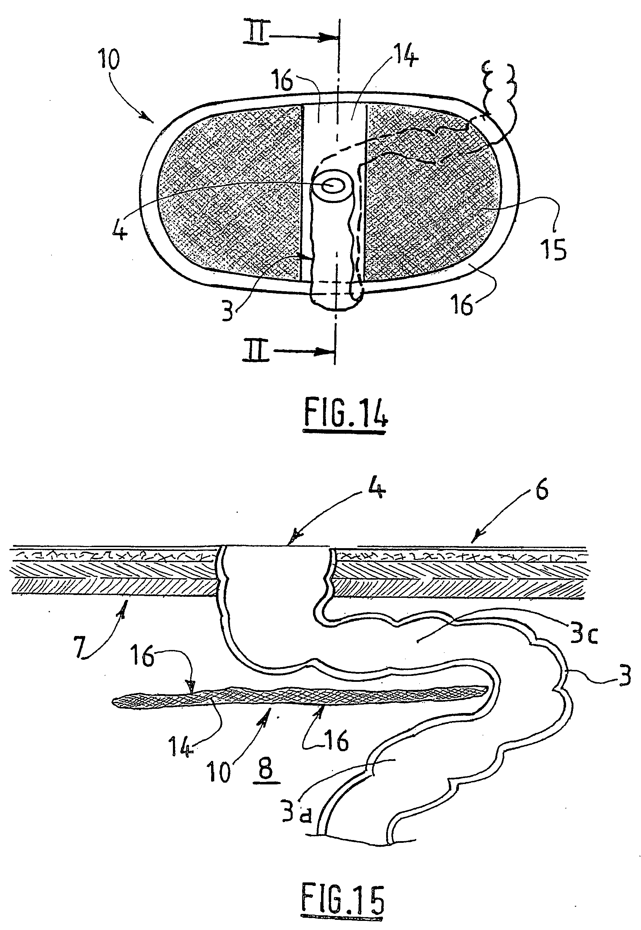

Referring to Figures 4 and 6, an implant 10 according to the invention is shown which comprises a layer of porous structure in the form of a biocompatible textile 11. As will be seen more clearly from Figures 13 and 15, the layer of porous structure or textile 11 comprises a first surface 12 intended to be placed facing the abdominal wall after implantation, and a second surface opposite the first surface 12, this second surface 13 being intended to be placed facing the abdominal cavity after implantation.

As will be seen clearly from Figure 4, which is a plan view of an implant according to the invention, the layer of porous structure comprises a first textile part 14 and a second textile part 15, the first textile part and the second textile part together forming the biocompatible textile 11 (see Figure 6). As will be seen more clearly from Figures 13-15 regarding the first surface 12 of the biocompatible textile 11, the first part 14 of textile is able to come into contact with the intestine, and the second part 15 of textile is intended to be placed facing the abdominal wall once the implant 10 according to the invention is implanted in the patient.

The implant 10 shown in Figure 4 is oval in shape. Its length can range, for example, from 15 to 30 cm, and its width can range, for example, from 12 to 20 cm. The shape of the implant can be adapted to the anatomy of the patient. It can also vary depending on the surgical technique envisaged.

In one example not shown, the implant has a generally round shape. Its diameter can then range from 5 to 20 cm, for example.

Referring to Figure 6, the implant 10 according to the invention is covered on its second surface 13 by a film 16 of anti-adhesive material. The edge 16a of the film of anti-adhesive material extends past the second surface 13 of the textile 11, for example by a distance ranging from 3 to 10 mm. Thus, the implant 10 is protected from contact with the viscera when it is implanted.

Figure 6 is a simplified cross-sectional view of the implant from Figure 4 along line II, where the thickness of the film 16 is exaggerated to make matters easier to understand. As will be seen clearly from Figure 6, the first part 14 of textile and the second part 15 of textile each have a thickness, namely a thickness E1 and a thickness E2, respectively. The value of the thickness E2 of the second part 15 of textile is superior to the value of the thickness E1 of the first part 14 of textile. Moreover, the film 16 completely encompasses the first part 14 of textile but only penetrates superficially into the thickness E2 of the second part 15 of textile. In Figure 6, the thickness of the film 16 is exaggerated. It must be understood that the film 16 penetrates into the second part 15 of textile only by a short distance, for example by a distance corresponding to 2 to 10% of the thickness E2. In the example shown, the value of the thickness E1 is 0.75 mm, while that of the thickness E2 is 2.00 mm.

Thus, as will be seen clearly from Figure 6, the first part 14 of textile is covered by film 16 of anti-adhesive material on its two surfaces, and this first part 14 of textile is totally enclosed within the film 16 of anti-adhesive material.

By contrast, as regards the second part 15 of textile, its first surface 12, intended to be placed facing the abdominal wall, is not covered by film 16 of anti-adhesive material. This surface 12 will be referred to hereinbelow as the open surface of the second part 15 of textile. By contrast, the second surface 13 intended to be placed facing the abdominal cavity, is covered by film 16 of anti-adhesive material. This surface 13 will be referred to hereinbelow as the closed surface of the second part of textile. Thus, the film 16 of anti-adhesive material penetrates only superficially into the second part 15 of textile, in the area of its closed surface 13, leaving open the porosity of the first open surface 12 of the second textile part 15.

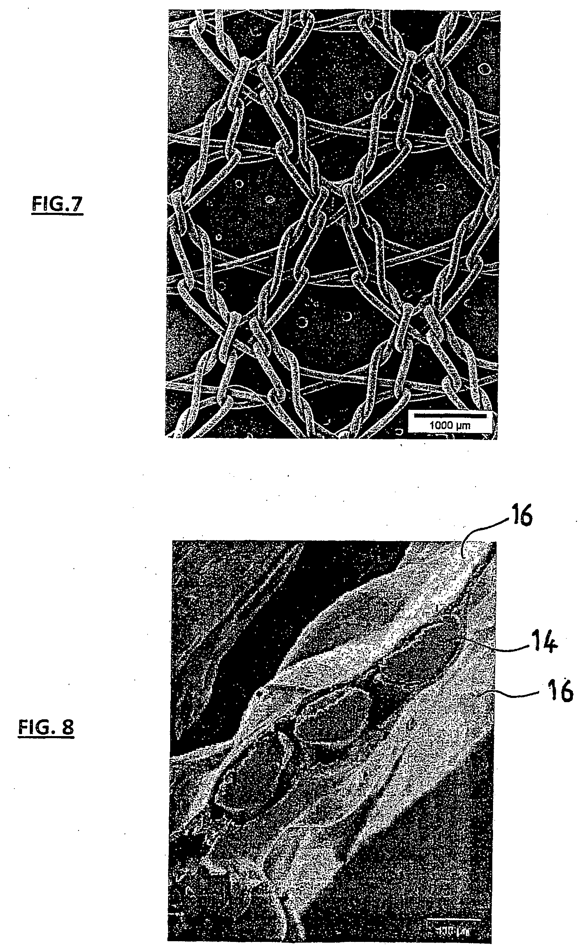

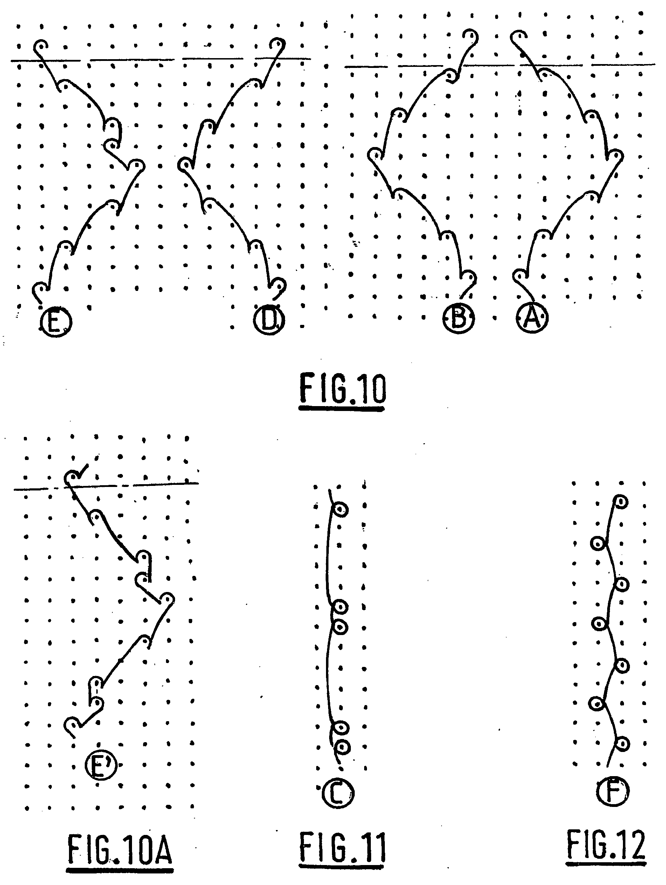

Figure 7 shows a view of the first part 14 of textile. In this example, the first part of textile is a knit obtained on a warp knitting machine or raschel machine with two guide bars A and B, threaded regularly with one guide full, one guide empty, using the knitting structure shown in Figure 10 for bars A and B. The respective charts used for bars A and B are the following:
Bar A: 4-4-5-4/4-4-4-3/3-3-2-1/1-1-0-1/1-1-1-2/2-2-3-4//
Bar B: 1-1-0-1/1-1-1-2/2-2-3-4/4-4-5-4/4-4-4-3/3-3-2-1//

The yarn used is preferably a monofilament yarn of polyethylene terephthalate, diameter 0.08 mm and titre 69 dtex. The knit thus formed comprises two opposite surfaces but is free of connecting sheets between its two opposite surfaces. It is a two-dimensional knit according to the present application.

The thickness of the first part of textile formed from such a knit is approximately 0.25 mm.

In the example shown, the knitting used for the first part of textile creates pores, preferably with dimensions that can range from 0.1 to 3 mm, preferably from 1.5 to 2 mm. At the moment of implantation, these pores are not visible, nor are they accessible to tissue colonization, because the whole of the first part of textile is confined in the film 16 of anti-adhesive material. However, after a few days, as the film of anti-adhesive material is absorbed and disappears after performing its function of limiting and/or avoiding formation of adhesions during the first 10 days following the implantation operation, the pores of the first part 14 of textile become accessible to tissue colonization. When a yarn of polyethylene terephthalate is used for producing the two-dimensional knit, this knit is non-bioabsorbable and remains permanently at the implantation site.

In another embodiment of the invention, said first part 14 of textile is made of a bioabsorbable material that is absorbed more slowly than the bioabsorbable material constituting the film 16 of anti-adhesive material.

As is shown in Figure 8, which is a scanning electron microscope photograph of a section of the implant according to one embodiment of the invention in the area of the first textile part, the latter is enclosed in the film 16 of anti-adhesive material. The coating of the first part 14 of textile by the film 16 of anti-adhesive material can be effected using any method known to a person skilled in the art. In the example shown in Figure 8, the first part 14 of textile is coated using the method described in the application WO2004/043294.

Thus, as will be seen clearly from Figure 8, the first part 14 of textile is covered by the film of anti-adhesive material on its two surfaces, and the porosity (see Figure 7) of the first part of textile is totally occluded at the moment of implantation. Thus, once covered with a film 16 of anti-adhesive material, the two surfaces of the first part 14 of textile are smooth and non-porous, as shown in Figure 8. The two surfaces of the first part 14 of textile do not damage the organs situated in the proximity of this first part 14 of textile, particularly the stoma organs.

The second part 15 of textile, of which the thickness is greater than that of the first part 14 of textile, can be a knit which is obtained on a warp knitting machine or double-bed raschel machine and which has two opposite surfaces connected to each other by connecting yarns, that is to say a three-dimensional knit according to the present application. For example, a first surface of the knit is produced with the two guide bars A and B already mentioned above for producing the first part 14 of textile, these being threaded identically and with the same charts as above. The second surface of the knit is produced with two supplementary guide bars D and E, threaded with one guide full, one guide empty, using the knitting structure shown in Figure 10 for bars D and E. The respective charts used for bars D and E are the following:
Bar D: 0-1-1-1/1-2-2-2/3-4-4-4/5-4-4-4/4-3-3-3/2-1-1-1//
Bar E: 5-4-4-4/4-3-3-3l2-1-1-1/0-1-1-1/1-2-2-2/3-4-4-4//

The connection of the two surfaces can be effected, for example, by hooking one loop in two, or in three, or in four, or in five, or in six of one of the bars D or E, whose knitting structure will be adapted. For example, in one embodiment of the invention, the connection of the two surfaces is effected by hooking one loop in three of the bar E, which thus becomes bar E', with the knitting structure shown in Figure 10A and according to the following chart:
Bar E': 5-4-3-4/4-3-3-3/2-1-1-1/0-1-2-1/1-2-2-2/3-4-4-4//

In another embodiment, the connection of the two surfaces can be effected with the aid of a fifth guide bar C, with the knitting structure shown in Figure 11 and according to the following chart:
Bar C: 1-0-1-0/1-1-1-1/1-1-1-1//

Thus, when the first part 14 of textile is in the form of a central strip separating two lateral strips of the second part 15 of textile, as is shown in Figures 4 and 5, the textile 11 can be produced in one piece, on the same knitting machine.
With the guide bars A, B, D and E' described above:
- the whole of the first surface 13 of the textile 11 is produced with the two guide bars A and B,
- along a first length, corresponding to the first lateral strip of the second part 15 of textile, the guide bars D and E' are threaded one guide full, one guide empty, in order to produce the second surface of the three-dimensional knit forming said second part 15 of textile,
- then, along the length corresponding to the width of the central strip of the first part 14 of textile, the guide bars D and E' are left empty in order to form the two-dimensional knit,
- finally, along a length corresponding to the second lateral strip of the second part 15 of textile, the guide bars D and E' are again threaded one guide full, one guide empty, in order to produce the second surface of the three-dimensional knit forming said second part 15 of textile.

In such a case, the optional fifth guide bar C is threaded only in the zones of the three-dimensional knit.

Finally, in order to obtain a smooth join between the three-dimensional knit forming the second part 15 of textile and the two-dimensional knit forming the first part 14 of textile, it is possible to use, still on the same knitting machine, a supplementary guide bar F in order to finish the edges of the three-dimensional knits, threaded in the area of these edges, according to the knitting structure shown in Figure 12, using the following chart for example:
Bar F: 1-0-1-1/1-2-1-1//

A monofilament yarn will preferably be chosen to produce the second part 15 of textile. This is because multifilament yarns pose greater risks of bacteria developing in the interstices present between the various filaments of the yarn.

The yarn used is preferably a monofilament yarn of polyethylene terephthalate, with a diameter of approximately 0.08 mm and titre 69 dtex.

The thickness of the second part 15 of textile, produced in the form of the three-dimensional knit described above, is approximately 1.50 mm.

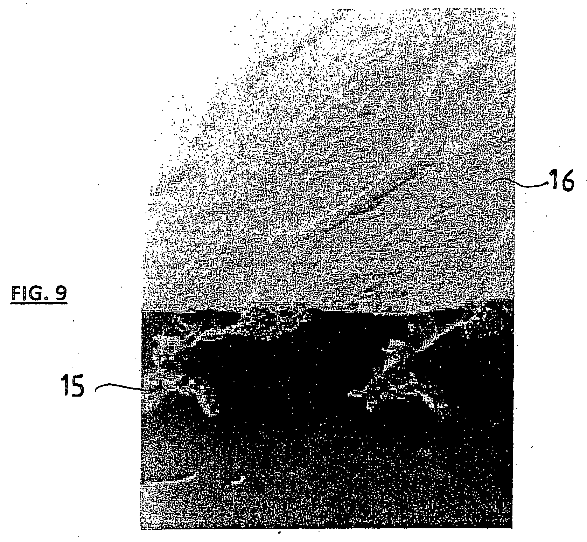

As will be seen from Figure 9, the second part 15 of textile is covered, on its surface intended to face the abdominal cavity, by the film 16 of anti-adhesive material. The film 16 of anti-adhesive material penetrates only superficially into the three-dimensional knit forming the second part 15 of textile. Consequently, the surface of the second part 15 of textile intended to face the abdominal wall is open, and its porosity is not occluded. This open surface therefore promotes all cellular growth.

The superficial covering of the surface of the second part 15 of textile intended to be placed facing the abdominal cavity can be carried out using any method known to a person skilled in the art, for example using the method described in the application WO99/06080.

The material used for the film 16 of anti-adhesive material can, for example, be collagen prepared in the manner described in the application WO99/06080.

The film 16 of anti-adhesive material may be applied to the surface of the textile 11 intended to be placed facing the abdominal cavity, in the following way:
- The solution of collagen is poured into a mould having the external dimensions desired for the film. The textile produced above is then applied to this solution, at the centre of the mould, the surface to be covered being placed on the solution of collagen. The solution of collagen then penetrates into the textile by capillary force, completely coating the first part of textile and covering the latter on the two opposite surfaces of the two-dimensional knit forming it, and penetrating only by a small distance into the thickness of the second part of textile, thus creating a superficial film for this three-dimensional part. Once the collagen has dried, the film is cut around the textile using a scalpel.

Alternatively, the covering/coating method described in WO2004/043294 can be used.

In another embodiment not shown here, the film 16 only superficially covers the surface of the first part of textile, intended to be placed facing the abdominal cavity, and does not encompass the two opposite surfaces of this first part of textile. In such a case, the surface of the first part of textile intended to be placed facing the abdominal wall is covered with a second film of anti-adhesive material. Thus, each of the two opposite surfaces of the first part of textile is covered by a smooth and continuous film of anti-adhesive material. Covering methods that can be used to form this second film are also described in WO2004/043294.

Figure 13 shows an implant according to the invention after it has been implanted, in the case of a direct stoma. To do this, the implant according to the invention shown in Figure 5 is used for example. In this figure, the reference numbers designating the same elements as in Figure 4 have been retained. The implant 10 in Figure 5 comprises an orifice 17 which has been created at the centre of the implant 10 and at the centre of the central strip formed by the first part 14 of textile. Such an orifice 17 can have a diameter ranging from 1 to 8 cm. A slit 18 starting from the central orifice 17 and opening out on an edge of the implant 10 allows the implant to be adjusted around the colon 3 during implantation of the implant.

In one embodiment not shown here, the orifice 17 is offset relative to the centre of the implant 10. It is also possible to have several orifices, depending on the surgery envisaged.

Thus, in Figure 13, an implant 10 similar to that in Figure 5 has been placed around the colon 3, which is at right angles to the abdominal wall 7 and to the skin 6. As will be seen from this figure, the first part 14 of textile covered entirely, that is to say on its two opposite surfaces, by the film 16 of anti-adhesive material is situated in direct proximity to the colon 3. Thus, the colon 3, which is a fragile organ, is not damaged by the implant 10. The open surface of the second part 15 of textile, which is porous and promotes cellular recolonization, is situated facing the abdominal wall 7. Thus, after implantation, the cells of the abdominal wall can gradually colonize the second part 15 of textile, for example the three-dimensional knit forming it.

It is possible to fix the implant 10 to the abdominal wall 7 using staples or sutures. In addition, or alternatively, the open surface of the second part 15 of textile can intrinsically comprise barbs or loops, which will facilitate its natural attachment to the abdominal wall. Such an affixing knit is described in the application WO01/81667.

Finally, the second surface of the textile, completely covered by film 16 of anti-adhesive material, is situated facing the abdominal cavity 8. Thus, the hollow and fragile organs, the viscera, are not damaged by the implant.

Figures 14 and 15 show an implant according to the invention after it has been implanted, in the case of an indirect stoma. To do this, the implant according to the invention in Figure 4 is used, for example. Figure 14 shows a plan view of the implant 10 according to Figure 4 at its implantation site in the area of the colon 3. For greater clarity, the skin and the abdominal wall have not been depicted. As will be seen from Figure 15, which is a cross-sectional view of Figure 14 along line II-II and in which the abdominal wall 7 and the skin 6 have been depicted, the colon 3 forms a bend prior to exteriorization, and the implant 10 is placed inside this bend. A part 3c of the colon is thus situated between the implant 10 and the abdominal wall 7.

As will be seen from these two figures, the part 3c of the colon faces and is able to come into contact with the first part 14 of textile covered on its two opposite surfaces by the film 16 of anti-adhesive material. Thus, neither the part 3c of the colon, situated between the implant 10 and the abdominal wall 7, nor the part 3d of the colon corresponding to the second length of the bend and able to lie under the implant 10 in the area of the abdominal cavity 8, risks being damaged by the implant 10. This is because the parts 3c and 3d of the colon 3 are each facing a surface of the first part 14 of textile covered by a film 16 of anti-adhesive material. Moreover, the relatively small thickness E1 of this first part 14 of textile permits flexible and atraumatic support of the colon 3.

In an indirect stoma of this kind, the implant 10 essentially acts like a hammock for the part 3c of the colon 3, and the implant 10 can be fixed to the abdominal wall 7 via the open surface of the second part 15 of textile placed facing the abdominal wall 7.

The implant according to the invention is used in particular in the treatment of parastomal hernias. It is able to support and/or protect the organs that are to be treated, such as the colon or ureters, without damaging them, while at the same time effectively strengthening the wall in which the stoma is formed, such as the abdominal wall, irrespective of the type of stoma formed, i.e. direct stoma or indirect stoma.

## Claims

1. Implant (10) for the prevention or treatment of a hernia formed in the abdominal wall (7) in the proximity of a stoma (4) of an organ (3), comprising a layer of a porous structure (11), said porous structure (11) comprising a first surface (12) intended to be placed facing the abdominal wall after implantation and a second surface (13) opposite the first surface (12), this second surface being intended to be placed facing the abdominal cavity after implantation, the second surface (13) intended to face the abdominal cavity (8) being covered by a first film (16) of anti-adhesive material, **characterized in that** said porous structure (11) comprises a first part (14) intended to be in contact with the stoma organ (3) and having a first thickness E1, and a second part (15) having a second thickness E2 greater than said first thickness E1, said first part (14) being covered, on its surface intended to face the abdominal wall (7), by a second film (16) of anti-adhesive material, the surface of the second part (15) intended to be placed facing the abdominal wall being not covered by a film of anti-adhesive material and remaining open to cell colonization at the time of implantation.

2. Implant (10) according to Claim 1, **characterized in that** the porous structure comprises a sponge, a fibrous matrix (11) or a combination of a sponge and of a fibrous matrix.

3. Implant (10) according to Claim 1 or 2, **characterized in that** the porous structure has pores with dimensions ranging from approximately 0.1 to approximately 3 mm.

4. Implant (10) according to Claim 2 or 3, **characterized in that** said porous structure comprises a textile (11).

5. Implant (10) according to any one of the preceding claims, **characterized in that** said first thickness E1 is from approximately 0.15 to 0.50 mm.

6. Implant (10) according to any one of the preceding claims, **characterized in that** said first part (14) of porous structure is a textile in the form of a two-dimensional knit.

7. Implant (10) according to any one of the preceding claims, **characterized in that** said second thickness E2 ranges from 0.40 to 3.00 mm.

8. Implant (10) according to any one of the preceding claims, **characterized in that** said second part (15) of porous structure is a textile in the form of a three-dimensional knit.

9. Implant (10) according to any one of the preceding claims, **characterized in that** said second part (15) of porous structure has, on its surface intended to face the abdominal wall (7), means of fastening said second part (15) to said abdominal wall (7).

10. Implant (10) according to Claims 6 and 8, **characterized in that** the two-dimensional knit (14) and the three-dimensional knit (15) are knitted together on the same knitting machine and constitute a textile (11) made in one piece.

11. Implant (10) according to any one of the preceding claims, **characterized in that** the anti-adhesive material constituting the first and or second film(s) is chosen from among the bioabsorbable materials, the non-bioabsorbable materials and their mixtures.

12. Implant (10) according to Claim 11, **characterized in that** the bioabsorbable materials suitable for the first and/or second film(s) of anti-adhesive material are chosen from among collagens, oxidized celluloses, polyarylates, trimethylene carbonates, caprolactones, dioxanones, glycolic acid, lactic acid, glycolides, lactides, polysaccharides, for example chitosans, polyglucuronic acids, hylauronic acids, dextrans and their mixtures.

13. Implant (10) according to Claim 11, **characterized in that** the non-bioabsorbable materials suitable for the first and/or second film(s) of anti-adhesive material are chosen from among polytetrafluoroethylene, polyethylene glycols, polysiloxanes, polyurethanes, stainless steels, derivatives of precious metals and their mixtures.

14. Implant (10) according to Claim 13, **characterized in that** the material constituting the first and/or second film(s) of anti-adhesive material is a hydrophilic bioabsorbable material, preferably chosen from the group formed by collagens, polysaccharides and their mixtures.

15. Implant (10) according to any one of the preceding claims, **characterized in that** said first film (16) of anti-adhesive material extends past the edges of the porous structure layer (11), for example by a distance that can range from 3 to 10 millimetres.

16. Implant (10) according to any one of the preceding claims, **characterized in that** the first film and the second film form a single and unique film (16), the first film then completely coating the first part (14) of porous structure and thus covering this first part of porous structure both on its surface intended to face the abdominal cavity and also on its surface intended to face the abdominal wall.

## Patentansprüche

1. Implantat (10) zur Verhütung oder Behandlung einer Hernie, die in der Bauchdecke (7) in der Nähe eines Stoma (4) eines Organs (3) ausgebildet ist, das eine Schicht aus einer porigen Struktur (11) umfasst, wobei die porige Struktur (11) eine erste Fläche (12) umfasst, die dazu bestimmt ist, nach der Implantation zu der Bauchdecke zeigend platziert zu sein, und eine zweite Fläche (13), die der ersten Fläche (12) entgegengesetzt ist, wobei diese zweite Fläche dazu bestimmt ist, nach der Implantation zu der Bauchhöhle zeigend platziert zu sein, wobei die zweite Fläche (13), die dazu bestimmt ist, zu der Bauchhöhle (8) zu zeigen, von einer ersten Folie (16) aus Antihaftmaterial bedeckt ist, **dadurch gekennzeichnet, dass** die porige Struktur (11) einen ersten Teil (14) umfasst, der dazu bestimmt ist, mit dem Stomaorgan (3) in Berührung zu sein und eine erste Stärke E1 hat, und einen zweiten Teil (15), der eine zweite Stärke E2 hat, die größer ist als die erste Stärke E1, wobei der erste Teil (14) auf seiner Fläche, die dazu bestimmt ist, zu der Bauchdecke (7) zu zeigen, durch eine zweite Folie (16) aus Antihaftmaterial bedeckt ist, wobei die Fläche des zweiten Teils (15), die dazu bestimmt ist, der Bauchdecke gegenüber platziert zu sein, nicht durch eine Folie aus Antihaftmaterial bedeckt ist und zur Zellbesiedlung im Zeitpunkt der Implantation offen bleibt.

2. Implantat (10) nach Anspruch 1, **dadurch gekennzeichnet, dass** die porige Struktur einen Schwamm, eine faserige Matrix (11) oder eine Kombination aus einem Schwamm und einer faserigen Matrix umfasst.

3. Implantat (10) nach Anspruch 1 oder 2, **dadurch gekennzeichnet, dass** die porige Struktur Poren mit Maßen hat, die von etwa 0, 1 bis etwa 3 mm reichen.

4. Implantat (10) nach Anspruch 2 oder 3, **dadurch gekennzeichnet, dass** die porige Struktur ein Textil (11) umfasst.

5. Implantat (10) nach einem der vorhergehenden Ansprüche, **dadurch gekennzeichnet, dass** die erste Stärke E1 etwa 15 bis 0,50 mm beträgt.

6. Implantat (10) nach einem der vorhergehenden Ansprüche, **dadurch gekennzeichnet, dass** der erste Teil (14) aus poriger Struktur ein Textil in der Form eines zweidimensionalen Gestricks ist.

7. Implantat (10) nach einem der vorhergehenden Ansprüche, **dadurch gekennzeichnet, dass** die zweite Stärke E2 von 0,40 bis 3,00 mm reicht.

8. Implantat (10) nach einem der vorhergehenden Ansprüche, **dadurch gekennzeichnet, dass** der zweite Teil (15) aus poriger Struktur ein Textil in der Form eines 3-dimensionalen Gestricks ist.

9. Implantat (10) nach einem der vorhergehenden Ansprüche, **dadurch gekennzeichnet, dass** der zweite Teil (15) aus poriger Struktur auf seiner Fläche, die dazu bestimmt ist, zu der Bauchdecke (7) zu zeigen, Mittel zum Befestigen des zweiten Teils (15) an der Bauchdecke (7) hat.

10. Implantat (10) nach den Ansprüchen 6 und 8, **dadurch gekennzeichnet, dass** das zweidimensionale (14) und das dreidimensionale Gestrick (15) auf derselben Strickmaschine zusammengestrickt werden und ein Textil (11), das aus einem Teil hergestellt ist, bilden.

11. Implantat (10) nach einem der vorhergehenden Ansprüche, **dadurch gekennzeichnet, dass** das Antihaftmaterial, das die erste und die zweite Folie bildet, aus den bioresorbierbaren Materialien, den nicht-bioresorbierbaren Materialien und ihren Gemischen ausgewählt ist.

12. Implantat (10) nach Anspruch 11, **dadurch gekennzeichnet, dass** die bioresorbierbaren Materialien, die für die erste und/oder die zweite Folie des Antihaftmaterials geeignet sind, aus Kollagenen, oxidierten Zellulosen, Polyacrylaten, Trimethylencarbonaten, Caprolactonen, Dioxanonen, Glykolsäure, Milchsäure, Glykoliden, Laktiden, Polysacchariden, zum Beispiel Chitosanen, Polyglucoronsäuren, Hyaluronsäuren, Dextranen und ihren Gemischen ausgewählt sind.

13. Implantat (10) Anspruch nach 11, **dadurch gekennzeichnet, dass** die Nicht-bioresorbierbaren Materialien, die für die erste und/oder zweite Folie Antihaftmaterials geeignet sind, aus Polytetrafluorethylen, Polyäthylenglykolen, Polysiloxanen, Polyurethanen, nicht rostenden Stählen, Derivaten von Edelmetallen und ihren Gemischen ausgewählt sind.

14. Implantat (10) nach Anspruch 13, **dadurch gekennzeichnet, dass** das Material, das die erste und/oder zweite Folie aus Antihaftmaterial bildet, ein hydrophiles bioresorbierbares Material ist, das vorzugsweise aus der Gruppe ausgewählt wird, die aus Kollagenen, Polysacchariden und ihren Gemischen besteht.

15. Implantat (10) nach einem der vorhergehenden Ansprüche, **dadurch gekennzeichnet, dass** sich die erste Folie (16) aus Antihaftmaterial über die Kanten der porigen Strukturschicht (11) hinaus erstreckt, zum Beispiel um eine Entfernung, die zwischen 3 und 10 mm liegen kann.

16. Implantat (10) nach einem der vorhergehenden Ansprüche, **dadurch gekennzeichnet, dass** die erste Folie und die zweite Folie eine einzige Folie (16) bilden, wobei die erste Folie dann den ersten Teil (14) aus poriger Struktur vollständig beschichtet und daher diesen ersten Teil aus porigen Struktur sowohl auf seiner Fläche, die dazu bestimmt ist, zu der Bauchhöhle zu zeigen, als auch auf seiner Fläche, die dazu bestimmt ist, zu der Bauchdecke zu zeigen, abdeckt.

## Revendications

1. Implant (10) destiné à prévenir ou traiter une hernie formée dans la paroi abdominale (7) à proximité d'une stomie (4) d'un organe (3) comprenant une couche d'une structure poreuse (11), ladite structure poreuse (11) comprenant une première surface (12) destinée à être placée pour faire face à la paroi abdominale après l'implantation et une deuxième surface (13) opposée à la première surface (12), cette deuxième surface étant destinée à être placée pour faire face à la cavité abdominale après l'implantation, la deuxième surface (13) destinée à faire face à la cavité abdominale (8) étant recouverte par un premier film (16) de matériau anti-adhésif, **caractérisé en ce que** ladite structure poreuse (11) comprend une première partie (14) destinée à être en contact avec l'organe de stomie (3) et ayant une première épaisseur E1, et une deuxième partie (15) ayant une deuxième épaisseur E2 supérieure à ladite première épaisseur E1, ladite première partie (14) étant recouverte, sur sa surface destinée à faire face à la paroi abdominale (7), par un deuxième film (16) de matériau anti-adhésif, la surface de la deuxième partie (15) destinée à être placée pour faire face à la paroi abdominale étant non recouverte par un film de matériau anti-adhésif et restant ouverte à la colonisation cellulaire au moment de l'implantation.

2. Implant (10) selon la revendication 1, **caractérisé en ce que** la structure poreuse comprend une éponge, une matrice fibreuse (11) ou une combinaison d'une éponge et d'une matrice fibreuse.

3. Implant (10) selon la revendication 1 ou 2, **caractérisé en ce que** la structure poreuse a des pores ayant des dimensions allant d'environ 0,1 à environ 3 mm.

4. Implant (10) selon la revendication 2 ou 3, **caractérisé en ce que** ladite structure poreuse comprend un textile (11).

5. Implant (10) selon l'une quelconque des revendications précédentes, **caractérisé en ce que** ladite première épaisseur E1 est d'environ 0,15 à 0,50 mm.

6. Implant (10) selon l'une quelconque des revendications précédentes, **caractérisé en ce que** ladite première partie (14) de la structure poreuse est un textile sous forme d'un tricot bidimensionnel.

7. Implant (10) selon l'une quelconque des revendications précédentes, **caractérisé en ce que** ladite deuxième épaisseur E2 va de 0,40 à 3,00 mm.

8. Implant (10) selon l'une quelconque des revendications précédentes, **caractérisé en ce que** ladite deuxième partie (15) de la structure poreuse est un textile sous forme d'un tricot tridimensionnel.

9. Implant (10) selon l'une quelconque des revendications précédentes, **caractérisé en ce que** ladite deuxième partie (15) de la structure poreuse a, sur sa surface destinée à faire face à la paroi abdominale (7), un moyen de fixation de ladite deuxième partie (15) de ladite paroi abdominale (7).

10. Implant (10) selon les revendications 6 et 8, **caractérisé en ce que** le tricot bidimensionnel (14) et le tricot tridimensionnel (15) sont tricotés ensemble sur la même machine à tricoter et constituent un textile (11) réalisé en un seul morceau.

11. Implant (10) selon l'une quelconque des revendications précédentes, **caractérisé en ce que** le matériau anti-adhésif constituant le premier et/ou le deuxième film(s) est choisi parmi les matériaux bioabsorbables, les matériaux non-bioabsorbables et leurs mélanges.

12. Implant (10) selon la revendication 11, **caractérisé en ce que** les matériaux bioabsorbables appropriés pour le premier et/ou le deuxième film(s) de matériau anti-adhésif sont choisis parmi les collagènes, les celluloses oxydées, les polyarylates, les carbonates de triméthylène, les caprolactones, les dioxannones, l'acide glycolique, l'acide lactique, les glycolides, les lactides, les polysaccharides, par exemple les chitosanes, les acides polyglucuroniques, les acides hyaluroniques, les dextranes et leurs mélanges.

13. Implant (10) selon la revendication 11, **caractérisé en ce que** les matériaux non-bioabsorbable appropriés pour le premier et/ou le deuxième film(s) de matériau anti-adhésif sont choisis parmi le polytétrafluoroéthylène, les polyéthylène glycols, les polysiloxanes, les polyuréthanes, les aciers inoxydables, les dérivés de métaux précieux et leurs mélanges.

14. Implant (10) selon la revendication 13, **caractérisé en ce que** le matériau constituant le premier et/ou le deuxième film(s) de matériau anti-adhésif est un matériau bioabsorbable hydrophile, de préférence choisi dans le groupe formé par les collagènes, les polysaccharides et leurs mélanges.

15. Implant (10) selon l'une quelconque des revendications précédentes, **caractérisé en ce que** ledit premier film (16) de matériau anti-adhésif s'étend au-delà des bords de la couche de structure poreuse (11), par exemple d'une distance qui peut varier de 3 à 10 millimètres.

16. Implant (10) selon l'une quelconque des revendications précédentes, **caractérisé en ce que** le premier film et le deuxième film forment un seul et unique film (16), le premier film enveloppant complètement la première partie (14) de la structure poreuse et couvrant ainsi cette première partie de la structure poreuse à la fois sur sa surface destinée à faire face à la cavité abdominale et aussi sur sa surface destinée à faire face à la paroi abdominale.
